(19) Europäisches Patentamt
European Patent Office
Office européen des brevets

(11) Veröffentlichungsnummer: **0 184 660**
**B1**

(12) **EUROPÄISCHE PATENTSCHRIFT**

(45) Veröffentlichungstag der Patentschrift: **06.04.88**

(51) Int. Cl.⁴: **C 07 C 147/06**, C 07 C 147/10

(21) Anmeldenummer: **85113961.8**

(22) Anmeldetag: **02.11.85**

(54) **Monocyclische Bis-oxethylsulfonyl-benzole und Verfahren zu ihrer Herstellung.**

(30) Priorität: **12.11.84 DE 3441274**

(43) Veröffentlichungstag der Anmeldung:
**18.06.86 Patentblatt 86/25**

(45) Bekanntmachung des Hinweises auf die Patenterteilung:
**06.04.88 Patentblatt 88/14**

(84) Benannte Vertragsstaaten:
**CH DE FR GB IT LI**

(56) Entgegenhaltungen:
**EP-A-0 092 909**
**DE-A-3 135 367**

**CHEMICAL ABSTRACTS, Band 101, Nr. 19, 5. November 1984, Seite 668, Spalte 1, Zusammenfassungsnr. 170876m, Columbus, Ohio, US**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher- Strasse 40, D-6000 Frankfurt am Main 50 (DE)**
Erfinder: **Arndt, Otto, Dr., Frankfurter Strasse 38, D-6238 Hofheim am Taunus (DE)**

EP 0 184 660 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Bis-oxethylsulfonylbenzole der allgemeinen Formel (1)

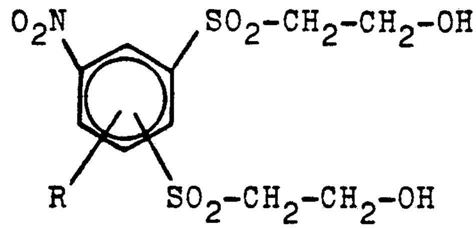

(1)

in welcher R ein Wasserstoff-, Chlor- oder Bromatom oder eine Alkyl$_{C_1-C_4}$ - oder Alkoxy$_{C_1-C_4}$ -Gruppe bedeutet und die beiden β-Oxethyl-sulfonyl-Gruppen in o- oder p-Stellung zueinander stehen, sowie ein Verfahren zu ihrer Herstellung, indem man (1) eine Verbindung der allgemeinen Formel (2)

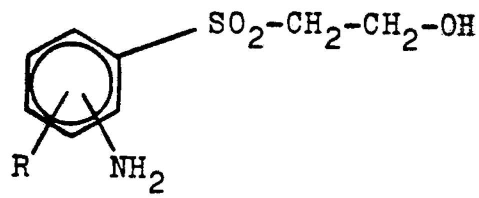

(2)

in welcher R die vorstehend genannte Bedeutung hat, und die Aminogruppe in o- oder p-Stellung zur -Oxethylsulfonyl-Gruppe steht, nach Überführung in ein Diazoniumhalogenid mit einer Mischung aus Alkalimetallhydrogensulfit und Halogenwasseratoffaäure in Gegenwart von Kupfer oder Kupferverbindungen in Abwesenheit oder in Gegenwart eines organischen Lösungsmittels bei Temperaturen von etwa 0°C bis etwa 50°C in das Sulfonsäurehalogenid der allgemeinen Formel (3)

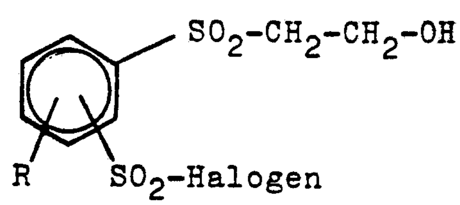

(3)

in welcher R die vorstehend genannte Bedeutung hat und die Sulfonsäurehalogenid-Gruppe in o- oder p-Stellung zur β-Oxyethylsulfonyl-Gruppe steht, überführt, (2) das so erhaltene Sulfonsäurehalogenid mit einem Alkalimetallsulfit in wäßrig-alkalischem Medium bei einem pH-Wert von etwa 6 bis 9 bei Temperaturen von etwa 20 - 90°C, vorzugsweise 40-70°C, in das entsprechende Alkalimetallsalz der Sulfinsäure der allgemeinen Formel (4)

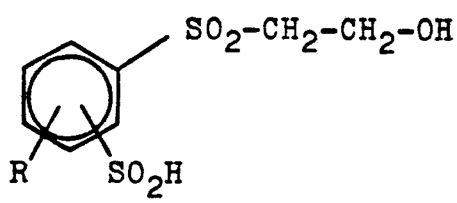

(4)

in welcher R die vorstehend genannte Bedeutung hat und die Gruppe -$SO_2H$ in o- oder p-Stellung zur β-Oxethylsulfonyl-Gruppe steht, überführt, (3) das so erhaltene Sulfinat in wäßrigem Medium mit Ethylenoxid bei einem pH-Wert von 6 bis 8 bei Temperaturen von etwa 20 bis etwa 150°C, vorzugsweise 50 - 120°-C, gegebenenfalls unter Druck, umsetzt zum Bisoxethylsulfonylbenzol der allgemeinen Formel (5)

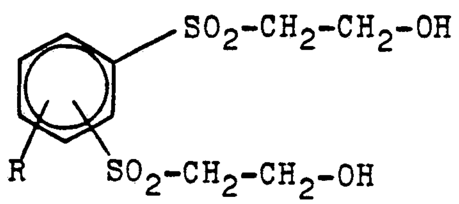

(5)

in welcher R die vorstehend genannte Bedeutung hat und die beiden β-Oxethylsulfonyl-Gruppen in o- oder p-Stellung zueinander stehen, (4) das so erhaltene Bis-oxethylsulfonylbenzol in an sich bekannter Weise in den entsprechenden Bis-schwefelsäurehalbester überführt und (5) letzteren mit einer mindestens stöchiometrischen Menge konzentrierter Salpetersäure, gegebenenfalls im Gemisch mit konzentrierter Schwefelsäure oder Oleum, bei Temperaturen von 20 bis 150°C zum Nitrophenyl-bis-oxethylsulfon-bis-schwefelsäurehalbester der Formel (6)

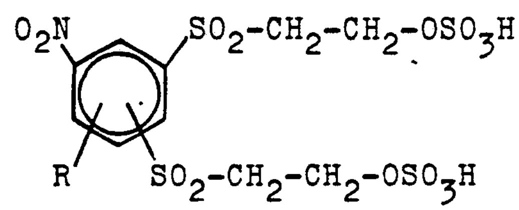

(6)

in welcher R die vorstehend genannte Bedeutung hat und die beiden β-Sulfatoäthylsulfonyl-Gruppen in o- oder p-Stellung zueinander stehen, nitriert und (6) letzteren durch Erhitzen der mit Wasser verdünnten Nitriermischung zur Verbindung der genannten Formel (1) hydrolysiert.

Bei der in erster Stufe vorgenommenen Überführung eines Diazoniumhalogenids der Verbindung der genannten Formel (2) in das entsprechende Sulfonsäurehalogenid der genannten Formel (3) wird zweckmäßigerweise das Diazoniumchlorid oder -bromid mit einer Mischung aus Salzsäure oder Bromwasserstoffsäure, vorzugsweise Salzsäure, und der technisch leicht zugänglichen Natriumbisulfitlauge, gegebenenfalls in Gegenwart eines organischen Lösungsmittels, vorzugsweise eines chlorierten aliphatischen Kohlenwasserstoffs, wie beispielsweise Methylenchlorid, Tetrachlorethan oder Trichlorethylen, umgesetzt, wobei die Reaktion in der Regel schon bei Raumtemperatur vor sich geht.

Die hierbei gebildeten Sulfonsäurehalogenide der genannten Formel (3) scheiden sich im allgemeinen schon im Verlauf der Reaktion in flüssiger oder fester Form ab und können beispielsweise durch Phasentrennung und ggf. Abdestillieren des zugesetzten, vorstehend genannten Lösungsmittels, oder durch Abfiltrieren, isoliert werden. In Beispiel 1 werden weitere Einzelheiten dieser Reaktionsstufe beschrieben. (Bei der in der DE-PS 2 308 262 prinzipiell beschriebenen Überführung von aromatischen Diazoniumhalogeniden in die entsprechenden aromatischen Sulfonsäurehalogenide unter Verwendung eines Alkalimetallhydrogensulfits in salzsaurer- oder bromwasserstoffsaurer Lösung in Gegenwart von Kupfer oder Kupferverbindungen wird in Abwesenheit organischer Lösungsmittel gearbeitet.)

Im Zuge der zweiten Reaktionsstufe (Überführung des Sulfonsäurehalogenids in das Sulfinat) stellt man die erforderliche wäßrige Alkalimetallsulfitlösung zweckmäßigerweise her aus Alkalimetallbisulfit und Alkalilauge, vorzugsweise Natriumbisulfit und Natronlauge. Die so hergestellte wäßrige Alkalimetallsulfitlösung wird zweckmäßigerweise im Temperaturbereich von 20 - 90°C, vorzugsweise 40 - 70°C, innerhalb 1 bis 5 Stunden, vorzugsweise 2 bis 4 Stunden, gleichzeitig mit dem Sulfonsäurehalogenid, vorzugsweise Sulfonsäurechlorid oder Sulfonsäurebromid, und der Alkalilauge, vorzugsweise Natronlauge, versetzt, wobei der pH-Wert zwischen etwa 6 und 9 gehalten wird. Die erhaltene wäßrige Lösung des Alkalimetallsulfinats wird, gegebenenfalls nach Klären von ungelösten Bestandteilen, ohne Zwischenisolierung mit Ethylenoxid bei Temperaturen von 20 bis 150°C, vorzugsweise 50 bis 120°C, gegebenenfalls unter Druck, umgesetzt, wobei durch gleichzeitige Zugabe von Mineralsäure ein pH-Wert von 6 bis 8 eingehalten wird. Das entstandene Bis-oxethylsulfonyl-benzol wird nach beendeter Umsetzung, gegebenenfalls nach Abkühlen und/oder Aussalzen, durch Filtration oder Extraktion isoliert.

Weitere Einzelheiten zur Herstellung von Sulfinat und Bisoxethylsulfonyl-benzol werden in Beispiel 2 angegeben.

Die so erhaltenen Bis-oxethylsulfonyl-benzole werden anschließend zur Nitrierung durch 2-bis 4-stündiges Verrühren mit der 3- bis 10-fachen Menge konzentrierter Schwefelsäure bei 20 bis 50°C quantitativ in eine schwefelsaure Lösung ihrer Bis-sulfatester überführt und diese durch Zulauf einer zumindest stöchiometrischen Menge konzentrierter Salpetersäure, gegebenenfalls im Gemisch mit konzentrierter Schwefelsäure oder Oleum, bei 20 bis 150°C zu den Nitrophenyl-bis-oxethylsulfon-bisschwefelsäurehalbestern umgesetzt, die als solche durch Verdünnen mit Wasser und Aussalzen isoliert, aber auch durch mehrstündiges Erhitzen der mit Wasser verdünnten Nitriermischungen auf Temperaturen von 80 -

120°C zu den freien Bis-oxethylsulfonyl-nitrobenzolen verseift und nach Abkühlen auf 0 - 20°C durch Filtration oder Extraktion in dieser Form gewonnen werden können.

Weitere Einzelheiten zur Veresterung, Nitrierung und Verseifung unter Erhalt der Endprodukte der Formel (1) werden in Beispiel 3 angegeben.

Die Herstellung der Ausgangsverbindungen der genannten allgemeinen Formel (2) erfolgt durch Verseifung der entsprechenden N-Acetylverbindungen (siehe Beispiel 1), die ihrerseits auf die in "Winnacker-Küchler, Chemische Technologie, Carl Hanser Verlag München, Wien 1982, Band 6, Seite 186", beschriebene Weise hergestellt werden können.

Die neuen Bis-oxethylsulfonyl-nitrobenzole der genannten allgemeinen Formel (1) stellen wertvolle Vorprodukte zur Herstellung von Reaktivfarbstoffen der Vinylsulfon-Reihe dar. Durch die mit diesen Verbindungen in den daraus hergestellten Farbstoffen erreichbare Erhöhung faserreaktiver Zentren kann eine bessere Farbstoff-Fixierung auf der Cellulosefaser (Baumwolle) und damit eine höhere Farbstoffausbeute und eine geringere Abwasserbelastung durch inaktiven Farbstoff erzielt werden. Die neuen Vorprodukte können beispielsweise nach Reduktion der Nitrogruppe zur primären Aminogruppe in an sich bekannter Weise, beispielsweise durch Reduktion mit Eisen/Säure oder katalytische Reduktion an Nickel- oder Edelmetallkatalysatoren (vgl. hierzu beispielsweise HOUBEN-WEYL, Bd. XI/1, S. 394-406/ Seiten 374-382) in wäßrigem Medium, als Diazokomponenten zur Herstellung wertvoller Reaktivfarbstoffe der Vinylsulfon-Reihe eingesetzt werden.

Die nachstehenden Beispiele sollen das erfindungsgemäße Verfahren näher erläutern, ohne es darauf zu beschränken.

**Beispiel 1**

243 Teile 4-Acetaminophenyl-oxethylsulfon werden in 1000 Teilen 7 %-iger Salzsäure durch Kochen am Rückfluß zum freien Amin verseift. Die abgekühlte Lösung wird mit 750 Teilen 30 %-iger Salzsäure und 300 Teilen Eis versetzt. Dann läßt man 183 Teile 40 %-ige Natriumnitrit-Lösung bei 5 - 10°C zulaufen. Nach Klärung der so hergestellten Diazolösung läßt man sie innerhalb von 3 Stunden gleichzeitig mit 183 Teilen wäßriger 40 %-iger Natriumbisulfit-Lösung unter die Oberfläche einer Mischung aus 960 Teilen 30 %-iger Salzsäure, 36 Teilen Kupfer(II)-sulfat. $5H_2O$, 183 Teilen wäßriger 40 %-iger Natriumbisulfit-Lösung und 2500 Teilen Methylenchlorid zulaufen, wobei die Temperatur allmählich von ca. 10 auf 20°C angehoben wird und Verluste an Schwefeldioxid zu vermeiden sind. Man rührt bei 20°C bis zum Ende der Stickstoffentwicklung (= ca. 30 1 unkorrigiert) nach. Man prüft auf den vollständigen Umsatz des Diazoniumsalzes durch Tüpfeln gegen R-Salzlösung. (R-Salz = Natriumsalz der 2-Naphthol-3,6-disulfonsäure).

Nach Trennen der organischen von der wäßrigen Phase wird die organische Phase über Natriumsulfat getrocknet, filtriert und im Vakuum das Methylenchlorid abdestilliert. Der flüssige Rückstand wird zur Kristallisation abgekühlt. Man erhält 200 Teile Benzoloxethylsulfon-4-sulfochlorid als ca. 85 %-iges Kristallisat (ermittelt aus dem Gehalt an verseifbarem Chlor) vom Schmelzpunkt 94 - 96°C. Die Ausbeute beträgt 70 % der Theorie.

**Beispiel 2**

285 Teile Benzoloxethylsulfon-4-sulfochlorid werden unter Rühren bei 25°C und einem pH von 8,0 in ca. 2 Stunden in ein Gemisch aus 335 Teilen wäßriger 40 %-iger Natriumhydrogensulfit-Lösung, 150 Teilen 33 %-iger Natronlauge und 1250 Teilen Wasser eingetragen. Der pH wird durch gleichzeitige Zugabe von zunächst ca. 30 Teilen Soda, dann ca. 200 Teilen 33 %-iger Natronlauge auf 8,0 gehalten. Man rührt 2 Stunden bei 40°C nach.

Dann stellt man mit ca. 30 Teilen 85 %-iger Phosphorsäure auf pH 6,0 und klärt bei 40°C über Kohle und Kieselgur. Man erhält 2340 Teile einer ca. 11 %-igen Lösung des Natriumsulfinates.

In diese Sulfinatlösung werden nach Verdünnen mit 800 Teilen Wasser bei pH 5,5 und 60°C in 2 Stunden 139 Teile Ethylenoxid gasförmig eingeleitet. Gleichzeitig werden ca. 400 Teile 20 %-ige Phosphorsäure pHgesteuert zudosiert, um den pH auf 5,5 zu halten. Man rührt bei 60°C bis zum Ende des Säurebedarfs nach (ca. 6 Stunden), währenddessen nochmals ca. 400 Teile 20 %-ige Phosphorsäure benötigt werden. Nach Austreiben von Spuren Ethylenoxid durch einen Stickstoffstrom (Absorption in 20 %-iger Schwefelsäure) wird die weiße Suspension auf 25°C abgekühlt und filtriert. Man erhält 177 Teile Benzol-1,4-bisoxethylsulfon als ca. 98 %-iges Kristallisat vom Schmelzpunkt 174-176°C mit der OH-Zahl = 374 (mg KOH/g), entsprechend einer Ausbeute von 60 % der Theorie, bezogen auf Benzoloxethylsulfon-4-sulfochlorid.

Die NMR-Spektren ($^1H$ und $^{13}C$) entsprechen der angegebenen Struktur.

**Beispiel 3**

45 Teile Benzol-1,4-bisoxethylsulfon werden durch Lösen in 300 Teilen 100 %-iger Schwefelsäure bei 40°C zum Bis-schwefelsäurehalbester umgesetzt. Dann läßt man 195 Teile Schwefeltrioxid als 65 %-ige Lösung in Schwefelsäure zulaufen. Die Lösung wird auf 100°C geheizt. Darauf werden bei 100°C 60 Teile 99 %-ige Salpetersäure in 2 Stunden zugetropft. Anschließend heizt man auf 140°C und hält noch 10 Stunden bei dieser Temperatur. Man läßt die Lösung in 5000 Teile Eis/Wasser-Gemisch einlaufen und kocht 5 Stunden am Rückfluß. Nach Neutralisation mit Natronlauge auf pH 7 dampft man im Vakuum zur Trockne ein, nimmt den Rückstand in N-Methylpyrrolidon auf, saugt noch heiß von den auskristallisierten anorganischen Salzen ab, regeneriert das N-Methylpyrrolidon durch Destillation im Vakuum, nimmt den Rückstand in Isopropanol heiß auf und filtriert erneut von anorganischen Salzen ab. Aus dem Filtrat isoliert man 46 Teile 2-Nitro-benzol-1,4-bisoxethylsulfon als ca. 98 %-iges Kristallisat vom Schmelzpunkt 156 - 157°C in einer Ausbeute von 89 % der Theorie.

**Beispiel 4**

169,5 Teile 2,5-Di-(β-hydroxyethylsulfonyl)-nitrobenzol werden in eine 85°C warme Aufschlämmung von 75 Teilen Eisenpulver in 400 Teilen Wasser und 1 Teil konzentrierter Salzsäure unter gutem Rühren eingetragen. Man rührt bei dieser Temperatur noch 30 Minuten nach, stellt sodann mit Natronlauge einen pH-Wert von 8 ein und saugt den Ansatz heiß ab. Beim Abkühlen des Filtrates kristallisiert das 2,5-Di-(β-hydroxyethylsulfonyl)-anilin aus, das hieraus zu etwa 100 Teilen isoliert werden kann.

61,8 Teile diser Anilin-Verbindung werden bei etwa 20°C in eine Mischung aus 200 Teilen Schwefelsäuremonohydrat und 25 Teilen 65 %-igem Oleum unter Rühren eingetragen; es wird noch 12 Stunden nachgerührt. Die erhaltene klare Lösung wird anschließend bei einer Temperatur unterhalb 5°C in 1000 Teile einer 25 %-igen wäßrigen Kaliumchlorid-Lösung langsam eingerührt. Man rührt noch drei Stunden nach und saugt das ausgefallene Produkt ab, wäscht es mit 25 %-iger wäßriger Kaliumchlorid-Lösung und trocknet es. Es wird ein kaliumchloridhaltiges Pulver erhalten, welches das 2,5-Di-(β-sulfatoethylsulfonyl)-anilin zu etwa 60 % enthält.

Eine Lösung von 45,9 Teilen (bezogen auf 100 %-ige Substanz) der Kaliumverbindung des 2,5-Di-(β-sulfatoethylsulfonyl)anilins in 400 Teilen Wasser wird mit 200 Teilen Eis und 5 Volumenteilen 96 %-iger Schwefelsäure versetzt. Man diazotiert durch langsame Zugabe von 10,2 Volumenteilen einer wäßrigen 5n-Natriumnitrit-Lösung innerhalb von 60 Minuten. Der Ansatz wird noch bei einer Temperatur zwischen 0 und 3°C bis zur vollständigen Diazotierung nachgerührt, undschließlich der noch geringe Nitritüberschuß, wie üblich, mit Amidosulfonsäure zerstört.

Zu dieser Diazoniumsalzlösung läßt man unter Rühren innerhalb 10 Minuten bei etwa 5°C eine wäßrige schwefelsaure Lösung aus 18,8 Teilen 3-Chlor-N,N-bis-(β-sulfatoethyl)-anilin einlaufen. Man stellt mit Natriumcarbonat oder vorzugsweise zur Bindung der Schwefelsäure und deren Ausfällung als Calciumsulfat mit Calciumcarbonat einen pH-Wert zwischen 2 und 2,2 ein und hält das Kupplungsgemisch noch 3 Stunden bei diesem pH-Wert. Sodann wird mit Natriumcarbonat oder vorzugsweise Calciumcarbonat ein pH-Wert von 5,5 eingestellt und das ausgefallene Calciumsulfat abgesaugt. Das Filtrat wird mit Kaliumchlorid in einer Menge, die 20 % des Volumens des Filtrates entspricht, unter Rühren versetzt; es wird 4 Stunden nachgerührt und das ausgefallene Produkt abgesaugt und getrocknet.

Es wird ein organgerotes Pulver erhalten, das neben Kaliumchlorid den reaktiven Monoazofarbstoff der Formel

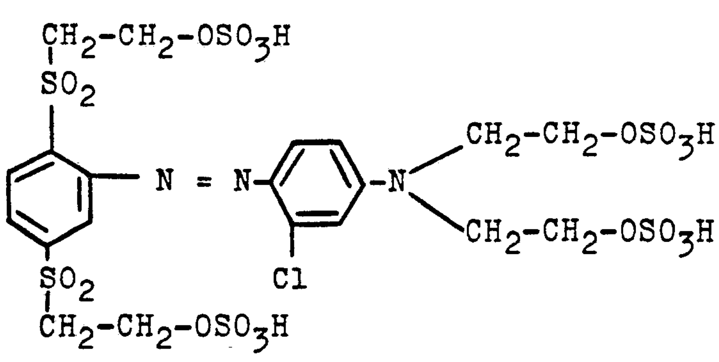

in Form des Kaliumsalzes enthält. Dieser Farbstoff, der in wäßriger Lösung ein $\lambda_{max}$ von 479 nm zeigt, besitzt sehr gute Farbstoffeigenschaften und eignet sich sehr gut zum Färben und Bedrucken von Cellulosefasermaterialien, wie Baumwolle. Unter Anwendung der für faserreaktive Farbstoffe üblichen Applikations- und Fixiermethoden liefert er mit hohem Fixiergrad farbstarke orangerote Färbungen und Drucke mit guten Echtheitseigenschaften, insbesondere Naßechtheiten, wie insbesondere guten Waschechtheiten bei

60 bis 95° C, guter alkalischer Schweißechtheit und Chlorwasserechtheit sowie guten Schweißlichtechtheiten.

**Beispiel 5**

111 Teile 3,4-Di-(β-hydroxyethylsulfonyl)-nitrobenzol werden in eine 85°C warme Aufschlämmung von 50 Teilen Eisenpulver in 350 Teilen Wasser und 1 Teil konzentrierter wäßriger Salzsäure unter gutem Rühren eingetragen. Man rührt bei dieser Temperatur noch 30 Minuten nach, stellt sodann mit Natronlauge einen pH-Wert von 8 ein und saugt den Ansatz heiß ab. Beim Abkühlen des Filtrates kristallisiert das 3,4-Di-(β-hydroxylethylsulfonyl)-anilin aus, das hieraus zu etwa 85 Teilen isoliert werden kann.

61,8 Teile dieser Anilin-Verbindung werden bei etwa 20°C in eine Mischung aus 200 Teilen Schwefelsäuremonohydrat und 25 Teilen 65 %-igem Oleum unter Rühren eingetragen; es wird noch 12 Stunden nachgerührt. Die erhaltene klare Lösung wird anschließend bei einer Temperatur unterhalb 5°C in 100 Teile einer 25 %-igen wäßrigen Kaliumchlorid-Lösung langsam eingerührt. Man rührt noch drei Stunden nach und saugt das ausgefallene Produkt ab, wäscht es mit 25 %-iger wäßriger Kaliumchlorid-Lösung und trocknet es. Es wird ein kaliumchloridhaltiges Pulver erhalten, welches das 3,4-Di-(β-sulfatoethylsulfonyl)-anilin zu etwa 60 % enthält.

Nach Diazotieren der so erhaltenen Anilinverbindung und Kuppeln des Diazoniumsalzes mit 3-Chlor-N,N-bis-(ß-sulfatoethylsulfonyl)-anilin analog Beispiel 4 erhält man ein gelbstichig rotes Pulver, das neben Kaliumchlorid den reaktiven Monoazofarbstoff der Formel

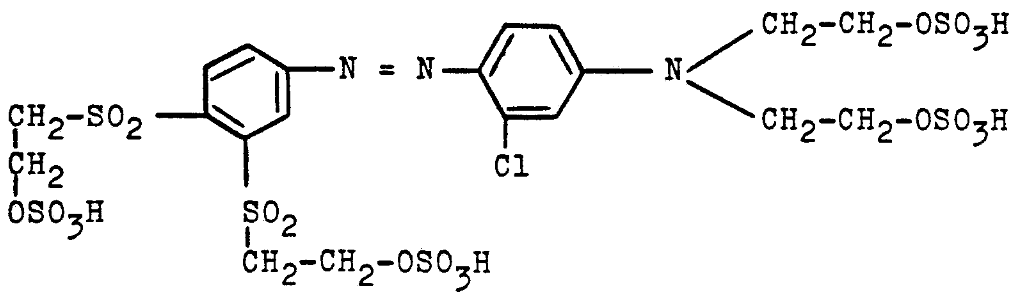

in Form des Kaliumsalzes enthält. Der Farbstoff, der in wäßriger Lösung ein $\lambda_{max}$ von 493 nm zeigt, besitzt sehr gute Farbstoffeigenschaften und eignet sich sehr gut zum Färben und Bedrucken von Cellulosefasermaterialien, wie Baumwolle. Unter Anwendung der für faserreaktive Farbstoffe üblichen Applikations- und Fixiermethoden liefert er mit hohem Fixiergrad farbstarke gelbstichig rote Färbungen und Drucke mit guten Echtheitseigenschaften, wie insbesondere guten Waschechtheiten bei 60 bis 95°C, guter alkalischer Schweißechtheit, guter Chlorwasserechtheit und guten Schweißlichtechtheiten.

**Patentansprüche**

1. Verbindungen der allgemeinen Formel

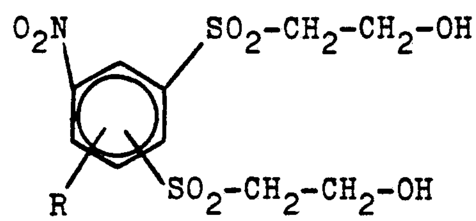

in welcher R ein Wasserstoff-, Chlor- oder Bromatom oder eine Alkyl$_{C_1-C_4}$- oder Alkoxy$_{C1-C4}$-Gruppe bedeutet und die beiden β-Oxethylsulfonyl-Gruppen in o- oder p-Stellung zueinander stehen.

2. Verbindung der Formel.

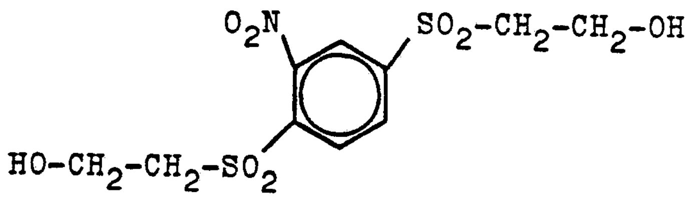

3. Verbindung der Formel

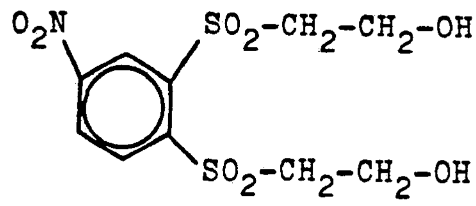

4. Verfahren zur Herstellung von Bis-oxethylsulfonylbenzolen der allgemeinen Formel (1)

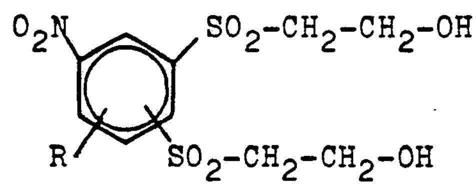

(1)

in welcher R ein Wasserstoff-, Chlor- oder Bromatom oder eine Alkyl$_{C_1\text{-}C_4}$-oder Alkoxy$_{C_1\text{-}C_4}$-Gruppe bedeutet, und die beiden β-Oxethylsulfonyl-Gruppen in o- oder p-Stellung zueinander stehen, dadurch gekennzeichnet, daß man eine Verbindung der allgemeinen Formel (2)

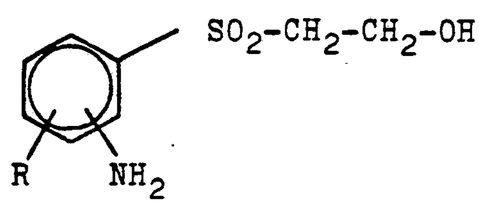

(2)

in welcher R die vorstehend genannte Bedeutung hat, und die Aminogruppe in o- oder p-Stellung zur β-Oxethylsulfonyl-Gruppe steht, nach Überführung in ein Diazoniumhalogenid mit einer Mischung aus Alkalimetallhydrogensulfit und Halogenwasserstoffsäure in Gegenwart von Kupfer oder Kupferverbindungen in Abwesenheit oder in Gegenwart eines organischen Lösungsmittels bei Temperaturen von etwa 0°C bis 50°C in das Sulfonsäurehalogenid der allgemeinen Formel (3)

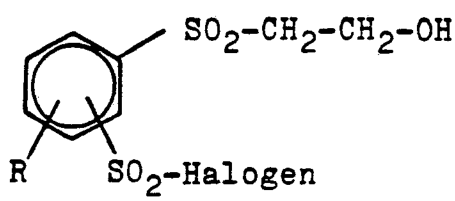

(3)

in welcher R die vorstehend genannte Bedeutung hat und die Sulfonsäurehalogenid-Gruppe in o- oder p-Stellung zur β-Oxyethylsulfonyl-Gruppe steht, überführt, (2) das so erhaltene Sulfonsäurehalogenid mit einem Alkalimetallsulfit in wäßrig-alkalischem Medium bei einem pH-Wert von etwa 6 bis 9 bei Temperaturen von etwa 20 - 90°C, in das entsprechende Alkalimetallsalz der Sulfinsäure der allgemeinen Formel (4)

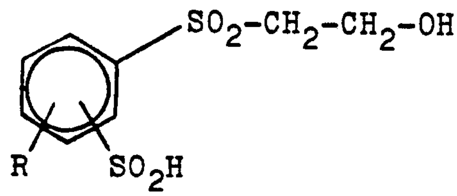

(4)

in welcher R die vorstehend genannte Bedeutung hat und die Gruppe-$SO_2H$ in o- oder p-Stellung zur β-Oxethylsulfonyl-Gruppe steht, überführt, (3) das so erhaltene Sulfinat in wäßrigem Medium mit Ethylenoxid bei einem pH-Wert von 6 bis 8 bei Temperaturen von etwa 20 bis etwa 150°C, vorzugsweise 50 - 120°C, gegebenenfalls unter Druck, umsetzt zum Bis-oxethylsulfonyl-benzol der allgemeinen Formel (5)

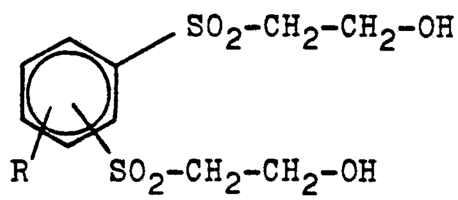

(5)

in welcher R die vorstehend genannte Bedeutung hat und die beiden β-Oxethylsulfonyl-Gruppen in o- oder p-Stellung zueinander stehen, (4) das so erhaltene Bis-oxethylsulfonyl-benzol in an sich bekannter Weise in den entsprechenden Bis-schwefelsäurehalbester überführt und (5) letzteren mit einer mindestens stöchiometrischen Menge konzentrierter Salpetersäure, gegebenenfalls im Gemisch mit konzentrierter Schwefelsäure oder Oleum, bei Temperaturen von 20 bis 150°C zum Nitrophenyl-bis-oxethylsulfon-bis-schwefelsäurehalbester der Formel (6)

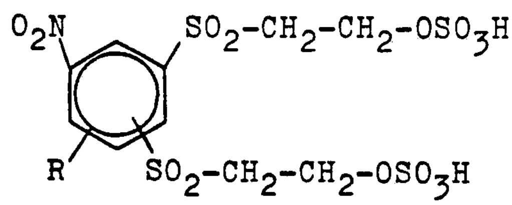

(6)

in welcher R die vorstehend genannte Bedeutung hat und die beiden β-Oxethylsulfonyl-Gruppen in o- oder p-Stellung zueinander stehen, nitriert und (6) letzteren durch Erhitzen der mit Wasser verdünnten Nitriermischung zur Verbindung der genannten Formel (1) hydrolysiert.

**Claims**

1. Compounds of the general formula

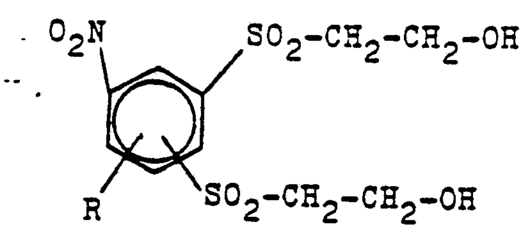

in which R denotes a hydrogen, chlorine or bromine atom or a $C_1$-$C_4$-alkyl or $C_1$-$C_4$-alkoxy group and the two β-hydroxyethylsulfonyl groups are in the o- position or p- position in relation to one another.

2. The compound of the formula

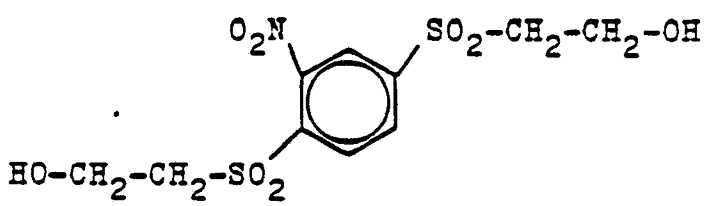

3. The compound of the formula

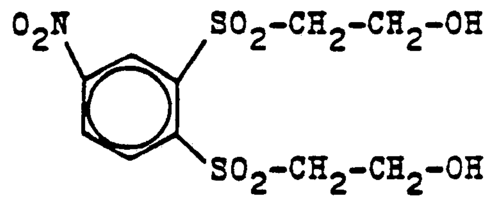

4. A process for the preparation of bishydroxyethylsulfonylbenzenes of the general formula (1)

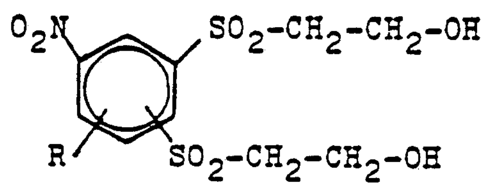

(1)

in which R denotes a hydrogen, chlorine or bromine atom or a $C_1$-$C_4$-alkyl or $C_1$-$C_4$ alkoxy group and the two β-hydroxyethylsulfonyl groups are in the o- position or p- position in relation to one another, which comprises (1) converting a compound of the general formula (2)

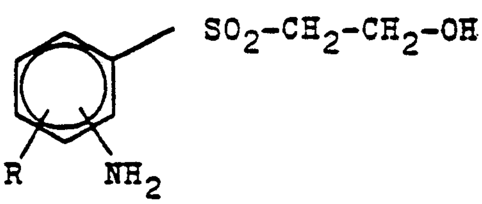

(2)

in which R has the meaning mentioned above and the amino group is in the o-position or p-position in relation to the β-hydroxyethylsulfonyl group, after conversion into a diazonium halide, into the sulfonyl halide of the general formula (3)

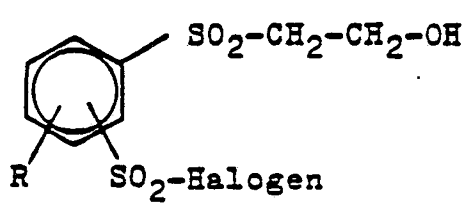

(3)

in which R has the meaning mentioned above and the sulfonyl halide group is in the o-position of p-position in relation to the β-hydroxyethylsulfonyl group, by means of a mixture of an alkali metal bisulfite and hydrogen halide acid in the presence of copper or copper compounds and in the absence or presence of an organic solvent at temperatures of about 0°C to about 50°C, (2) converting the sulfonyl halide thus obtained into the corresponding alkali metal salt of the sulfinic acid of the general formula (4)

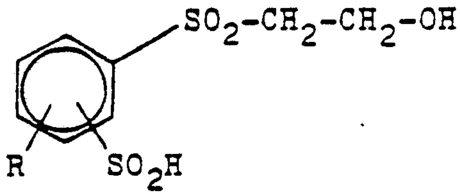

(4)

in wich R has the meaning mentioned above and the $-SO_2H$ group is in the o-position or p-position in relation to the β-hydroxyethylsulfonyl group, by means of an alkali metal sulfite in an aqueous alkaline medium at a pH of about 6 to 9 and at temperatures of about 20 - 90° C, (3) reacting the sulfinate thus obtained in an aqueous medium with ethylene oxide at a pH of 6 to 8 and at temperatures of about 20 to about 150° C, preferably 50 - 120° C, and if necessary under pressure, to give the bishydroxyethylsulfonylbenzene of the general formula (5)

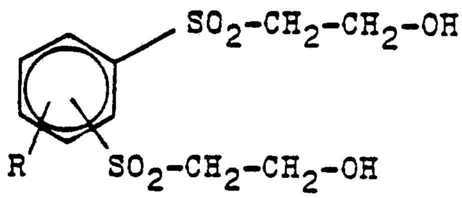

(5)

in which R has the meaning mentioned above and the two β-hydroxyethylsulfonyl groups are in the o-position or p-position in relation to one another, (4) converting the bishydroxyethylsulfonylbenzene thus obtained into the corresponding bis-sulfuric acid half ester in a manner known per se and (5) nitrating the latter with at least a stoichiometric amount of concentrated nitric acid, if appropriate as a mixture with concentrated sulfuric acid or oleum, at temperatures of 20 to 150° C, to give the nitrophenylbishydroxyethylsulfonylbis-sulfuric acid half ester of the formula (6)

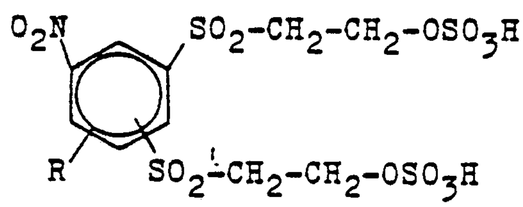

(6)

in which R has the meaning mentioned above and the two β-sulfatoethylsulfonyl groups are in the o-position or p-position in relation to one another, and (6) hydrolizing the latter by heating the nitration mixture after dilution with water to give the compound of the formula (1) mentioned.

**Revendications**

1. Composés répondant à la formule générale:

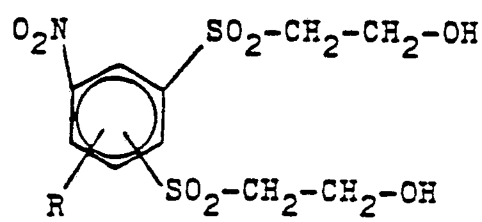

dans laquelle R représente un atome d'hydrogène, de chlore ou de brome, un radical alkyle en $C_1$-$C_4$ ou un radical alcoxy en $C_1$-$C_4$, et les deux radicaux hydroxy-2 éthylsulfonyles sont en position ortho ou para l'un par rapport à l'autre.

2. Composé de formule:

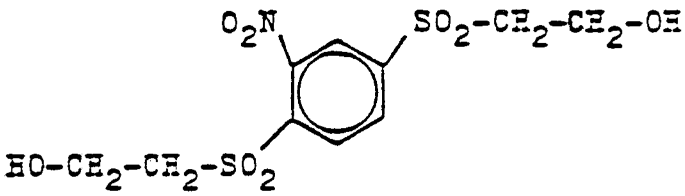

3. Composé de formule:

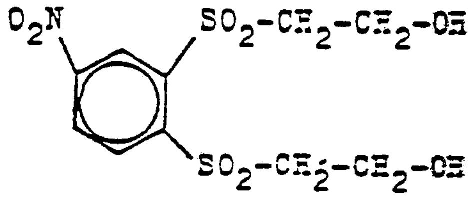

4. Procédé pour préparer des bis-(hydroxyéthylsulfonyl)-benzènes répondant à la formule générale (1):

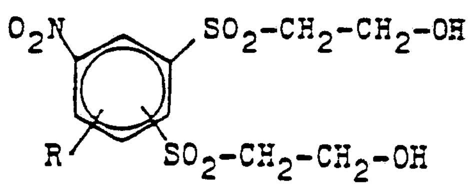

(1)

dans laquelle R représente un atome d'hydrogène, de chlore ou de brome, un radical alkyle en $C_1$-$C_4$ ou un radical alcoxy en $C_1$-$C_4$, et les deux radicaux hydroxy-2 éthylsulfonyles sont en position, ortho ou para l'un par rapport à l'autre, procédé caractérisé en ce que:

1° - on transforme un composé répondant à la formule générale (2):

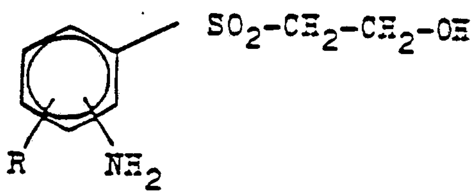

(2)

dans laquelle R a la signification précédemment donnée et le radical amino se trouve en position ortho ou para relativement au radical hydroxy-2 éthyl-sulfonyle, après l'avoir converti en un halogénure de diazonium, au moyen d'un mélange d'un hydrogénosulfite de métal alcalin et d'un acide halohydrique, en présence de cuivre ou de composés du cuivre, en présence ou non d'un solvant organique, à des températures comprises entre environ 0 et environ 50° C, en l'halogénure de sulfonyle répondant à la formule générale (3):

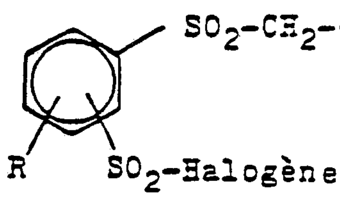

(3)

dans laquelle R a la signification précédemmerit donnée et le radical halogénosulfonyle se trouve en position ortho ou para relativement au radical l'hydroxy-2 éthylsulfonyle,

2° - on transforme l'halogénure de sulfonyle ainsi obtenu, au moyen d'un sulfite de métal alcalin, dans un milieu aqueux alcalin, à un pH d'environ 6 à 9 et à des températures d'environ 20 à 90° C, en le sel de métal alcalin correspondant de l'acide sulfinique répondant à la formule générale (4):

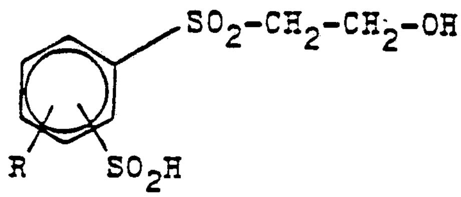

(4)

dans laquelle R a la signification précédemment donnée et le radical $-SO_2H$ se trouve en position ortho ou para relativement au radical hydroxy-2 éthylsulfonyle,

3° - on fait réagir le sulfinate ainsi obtenu, en milieu aqueux, avec l'oxyde d'éthylène à un pH de 6 à 8 et à des températures d'environ 20 à environ 150° C, de préférence de 50 à 120° C, éventuellement sous pression, de manière à obtenir le bis-(hydroxy-éthylsulfonyl)-benzène répondant à la formule générale (5):

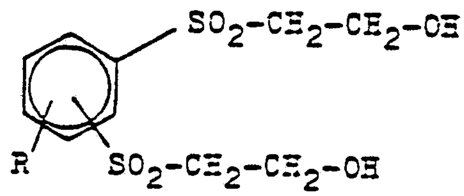

(5)

dans laquelle R a la signification précédemment donnée et les deux radicaux hydroxy-2 éthylsulfonyles se trouvent en position ortho ou para l'un par rapport à l'autre,

4° - on transforme le bis-(hydroxy-éthylsulfonyl)-benzène ainsi obtenu, de manière connue, en le bis-(hémi-ester sulfurique) correspondant,

5° - on nitre ce dernier avec une quantité d'acide nitrique concentré au moins égale à la quantité stoechiométrique, éventuellement en mélange avec de l'acide sulfurique concentré ou de l'oléum, à des températures de 20 à 150° C, de manière la obtenir le nitro-bis-(sulfatoéthylsulfonyl)-benzène répondant à la formule (6):

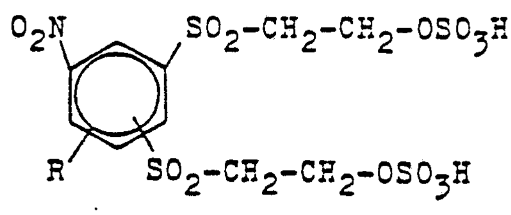

(6)

dans laquelle R a la signification précédemment donnée et les deux radicaux sulfato-2 éthylsulfonyles se trouvent en position ortho ou para l'un par rapport à l'autre, et

6° - on hydrolyse ce dernier, en chauffant le mélange de nitration dilué à l'eau, pour le convertir en le composé de formule (1).